# EUROPEAN PATENT APPLICATION

(11) **EP 4 822 023 A1**
(43) Date of publication of application: **14.10.2026**
(21) Application number: 25169431.1
(22) Date of filing: 09.04.2025
(51) Int. Cl.: C12N 15/115

(54) **DOXYCYCLIN-BINDING, REGULATORY APTAMER**

(71) Applicant: Technische Universität Darmstadt, 64289 Darmstadt (DE)
(72) Inventor: Süß, Beatrix, 64287 Darmstadt (DE); Hötzel, Janis, 64283 Darmstadt (DE); Schäfer, Max, 64291 Darmstadt (DE)
(74) Representative: Fuchs Patentanwälte Partnerschaft mbB

(57) **Abstract**

The present disclosure relates to a novel class of nucleic acid molecules, specifically aptamers, that exhibit high affinity and specificity for doxycycline, a widely used antibiotic. These aptamers undergo a conformational change upon binding to doxycycline, enabling their application in a variety of biotechnological and therapeutic contexts. Aptamers are short, single-stranded nucleic acid sequences that can bind to target molecules with high selectivity, much like antibodies, but with distinct advantages such as smaller size, ease of synthesis, and the ability to be engineered for specific functions. Further disclosed are uses, pharmaceutical compositions and medical uses of such nucleic acid molecules.

## Description

The present disclosure relates to a novel class of nucleic acid molecules, specifically aptamers, that exhibit high affinity and specificity for doxycycline, a widely used antibiotic. These aptamers undergo a conformational change upon binding to doxycycline, enabling their application in a variety of biotechnological and therapeutic contexts. Aptamers are short, single-stranded nucleic acid sequences that can bind to target molecules with high selectivity, much like antibodies, but with distinct advantages such as smaller size, ease of synthesis, and the ability to be engineered for specific functions. Further disclosed are uses, pharmaceutical compositions and medical uses of such nucleic acid molecules.

### Background of the Disclosure

Aptamers are short, single-stranded nucleic acid molecules, either RNA or DNA, that can bind specifically to a target molecule, such as proteins, small molecules, or even cells. They are often referred to as "chemical antibodies" because, like antibodies, they bind with high affinity and specificity, but they are much smaller and can be synthesized more easily. Aptamers are typically selected through a process called SELEX (Systematic Evolution of Ligands by Exponential Enrichment), which involves the iterative selection of nucleic acids that bind tightly to a particular target. Upon binding, aptamers often undergo a conformational change, which can be utilized for various applications, such as biosensors or gene regulation systems. Due to their versatility, aptamers are used in diagnostics, therapeutics, and drug delivery, among other fields.

Aptamers that bind to substances, for example such as tetracycline or doxycycline, and undergo conformational changes, have been explored and can be used in applications like riboswitches and aptazymes. Tetracycline and doxycycline are antibiotics that can bind to specific RNA or DNA aptamers, causing them to adopt different conformations. These conformational changes are often used as a mechanism to control gene expression or catalyze specific reactions.

For riboswitches, the aptamer region binds to doxycycline or tetracycline, triggering a conformational change that can influence the expression of a downstream gene. This change can either inhibit or activate gene expression in response to the presence of the antibiotic. Riboswitches that respond to tetracycline or doxycycline have been developed to control gene expression in bacterial systems, and they function similarly in synthetic biology applications.

The use of doxycycline-binding riboswitches offers several advantages. They allow precise control over gene expression, as the level of expression can be finely tuned by adjusting the concentration of doxycycline. This provides a high degree of temporal and spatial control. Additionally, regulation is non-invasive, as doxycycline can be administered orally or in other non-intrusive ways. Importantly, the regulatory effect is reversible, meaning gene expression can return to its baseline state once doxycycline is removed. These riboswitches are highly versatile and can be applied in various biological systems, including bacteria, yeast, and mammalian cells. Furthermore, their synthetic design allows them to be tailored for specific ligands, which opens up possibilities for highly customized gene regulatory systems.

In aptazymes, the aptamer's binding to doxycycline or tetracycline triggers a conformational change that activates the ribozyme's catalytic function, leading to a specific biochemical reaction. For example, an aptazyme could be engineered to cleave a specific RNA sequence or catalyse another reaction when doxycycline is present, making it useful for applications in gene regulation and synthetic biology.

These systems offer a high degree of specificity and control and are being increasingly explored in synthetic biology for their potential to regulate cellular functions in response to antibiotics like doxycycline and tetracycline.

A key feature of the aptamers described in this disclosure is their ability to bind doxycycline with high affinity, resulting in a significant conformational shift in the structure of the aptamer upon ligand binding. This conformational change can be exploited in several innovative applications, such as riboswitches, aptazymes and splice-regulators, which are molecular devices capable of regulating gene expression or catalyzing specific reactions in response to the presence of doxycycline.

In addition to their use in gene regulation and synthetic biology, these doxycycline-binding aptamers have potential applications in biosensors for detecting the presence of doxycycline in various environments, such as in clinical diagnostics or environmental monitoring. Their specificity and sensitivity make them ideal candidates for developing highly selective diagnostic tools and therapeutic agents.

The disclosure encompasses the design, synthesis, and applications of these doxycycline-binding aptamers, providing new possibilities for their use in molecular biology, drug delivery, gene regulation, and other fields where precise molecular recognition and control are required. The versatility of these aptamers opens the door to a broad range of applications in biotechnology, pharmaceutical development, and diagnostics, making them valuable tools for advancing research and healthcare.

### Brief Summary of the Disclosure

In a first aspect the disclosure relates to a nucleic acid molecule comprising a sequence that undergoes a conformational change upon binding of doxycycline, wherein the nucleic acid molecule comprises a sequence selected from the group of sequences according to SEQ ID NO: 1 to SEQ ID NO: 28.

In a second aspect the disclosure relates to the uses of the nucleic acid molecules as disclosed herein.

In a third aspect the disclosure relates to a pharmaceutical composition comprising the nucleic acid molecules as disclosed herein.

In a fourth aspect the disclosure relates to the nucleic acid molecules disclosed herein, or the pharmaceutical compositions disclosed herein, for use as a medicament.

### Description of the Figures

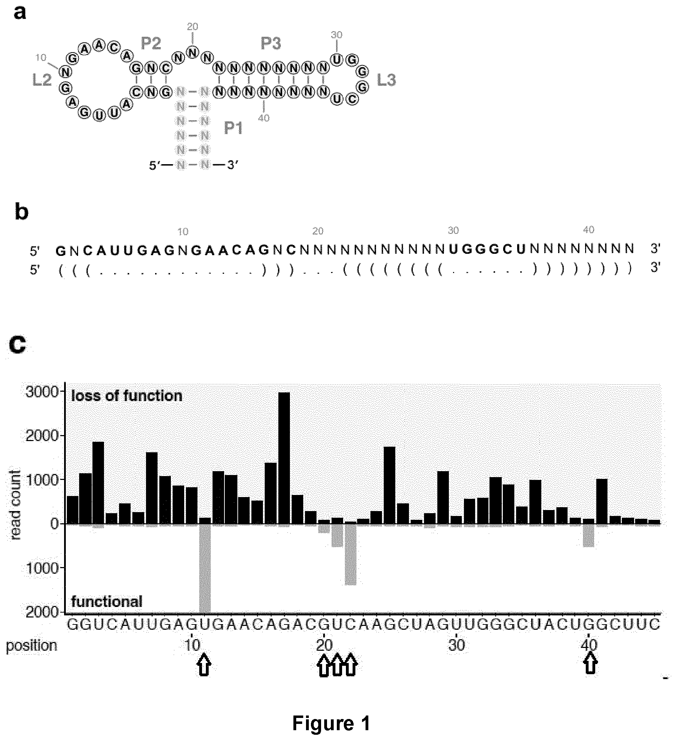

**Figure 1** - 1a) Two-dimensional representation of the aptamer with its overall structure (Seq. ID 1). Variable positions are shown as "N" while position allowing for no variability are presented with the exact nucleotide (A/U/C/G). The P1 stem is shown in grey, due to its stabilizing nature and its role for the regulation in yeast. Nevertheless, it is not important for the function of the aptamer and can be altered freely. 1b) Representation of the aptamer in nucleotide sequence and dot-bracket annotation of its structure, representing the above shown aptamer structure. 1c) Single nucleotide position variability tested by a randomized mutation screening. The x-axis represents the single nucleotide positions of the randomized part of the aptamer variant M10 (Seq. ID 13), while the y-axis shows the number of reads containing mutations, either leading to loss of function or a functional aptamer. Positions with a higher number of mutations leading to no loss of function, indicating variability of this position, are highlighted with an arrow.

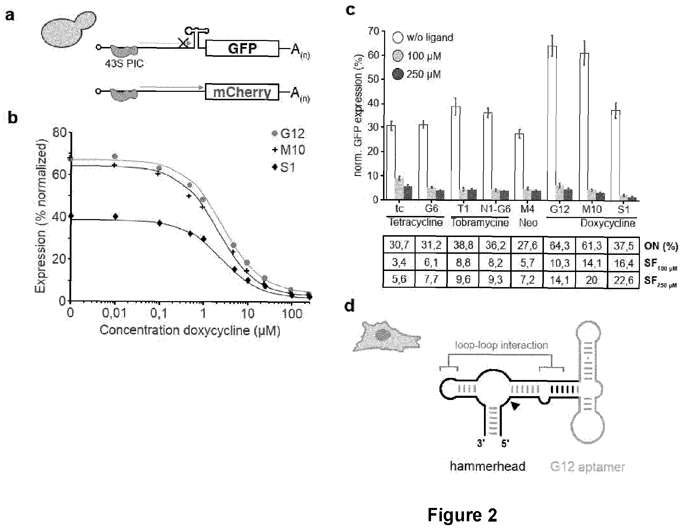

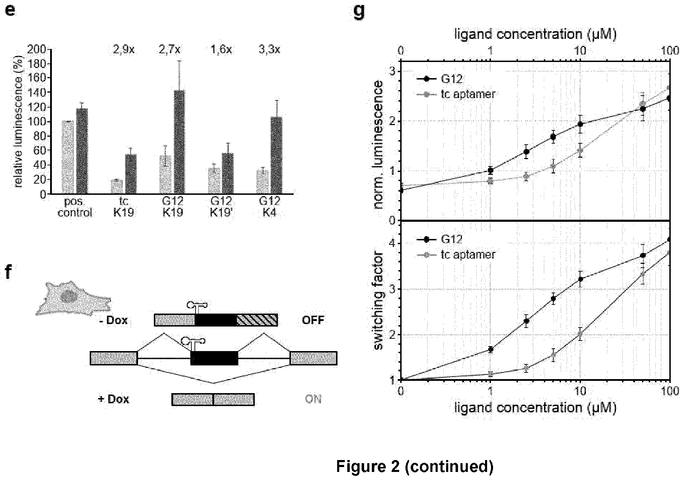

**Figure 2** - 2a) Schema of the *in vivo* testing of regulatory aptamers in yeast using the "roadblock mechanism". The aptamer candidates or mutants are cloned into the 5'UTR of a GFP gene. After transcription, the aptamer is located right before the Kozak sequence on the mRNA. When the 43S pre-initiation complex (PIC) of the ribosome is recruited to the mRNA it scans for the start codon, thereby having to pass the aptamer sequence. In the unbound state without the ligand, the aptamer is rather loose in its structure, allowing for easy readthrough of the PIC. Upon ligand binding regulatory aptamers undergo structural rearrangement. This keeps the PIC from reaching the start codon, thereby inhibiting translation of the downstream GFP coding sequence. 2b) Measurement of dose dependent response of the doxycycline aptamer G12 (grey dot, Seq. ID 3) and its variants M10 (black cross, Seq. ID 13) and S1 (black diamond) in yeast. Plotted on the x-axis are the added concentration of doxycycline in logarithmic form and on the y-axis the % of expression normalized to positive control. Different versions of the P1 stem in the three variants lead to different gene-regulatory behaviour: G12 and M10 possess a similar "ON" state without doxycycline with around 65% expression, whereas S1 possesses lower expression levels in the "ON" state with around 40% expression. All three variants show the same first response to doxycycline at 100 nM, have their EC50 around 5 µM and reach a plateau of lowest expression levels around 250 µM. 2c) Comparison of regulatory potential in yeast of the doxycycline-binding aptamer G12 (Seq. ID 3) and its variants M10 (Seq. ID 13) and S1 with previously published regulatory aptamers binding to tetracycline, tobramycin and neomycin. Each aptamer was tested without its ligand, with 100 µM and 250 µM ligand concentration. Below the graph, a table shows the expression levels in the "ON" state (without ligand) and the "switching factor" for 100 and 250 µM ligand. All aptamers show the highest switching at 250 µM ligand concentration, while the doxycycline-binding aptamers showing the best regulatory effect with "switching factors" ranging from 14,1 (G12) to 22,6 (S1) whereas the highest switching factor of another aptamer is 9,6 achieved by the T1 tobramycin aptamer. 2d) Schema of the hammerhead aptazyme. The aptamer is fused onto a stem of the aptazyme thereby regulating the inner-molecular interactions necessary for aptayzme activity. 2e) Gene regulation of aptazymes measured with a dual-luciferase assay in human HeLa cells. The y-axis plots the expression levels normalized to the positive control. The already tetracycline-aptazyme K19 shows a "switching factor" of 2,9-fold with a "ON" state of around 50%. When testing the doxycycline aptamer G12 in a hammerhead aptazyme, the resulting aptazyme showed the best gene regulation with variant K4. This construct achieved a "switching factor" of 3,3-fold with a "ON" state of around 100%. 2f) Schema of the splicing-regulating aptamer construct. Into the sequence of a gene, an artificial exon is introduced that contains stop codons which lead to premature termination of translation when the artificial exon is included into the mRNA. The 3' splice site (3'ss) of the artificial exon is placed in the basal stem of the aptamer, allowing for ligand-dependent accessibility of the splice site. When the ligand is added, the basal stem of the aptamer is stabilized, reducing the accessibility of the 3'ss, leading to exclusion of the artificial exon. In the absence of the ligand, the 3'ss is recognized and the artificial exon is included into the final mRNA, thereby reducing expression of the target gene. 2g) Dose response measurement of the splicing regulating aptamer constructs of the tetracycline- and the doxycycline-binding aptamer in human HeLa cells. The upper graph depicts the normalized luminescence on the y-axis of a dual-luciferase, where the aptamer-controlled luminescence of the Firefly luciferase is normalized to the constitutive expressed Renilla luciferase. The lower graph depicts the resulting "switching factor" of the measurements in x-fold switching on the y-axis. For both graphs the x-axis plots the doxycycline concentration. The doxycycline-binding aptamer G12 (black) shows the higher "switching factor" when compared to the tetracycline-binding aptamer (grey) and the earlier response to its ligand.

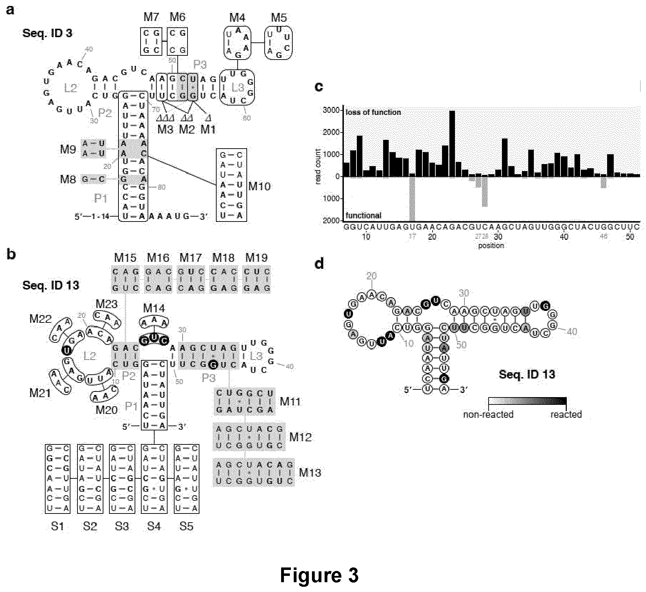

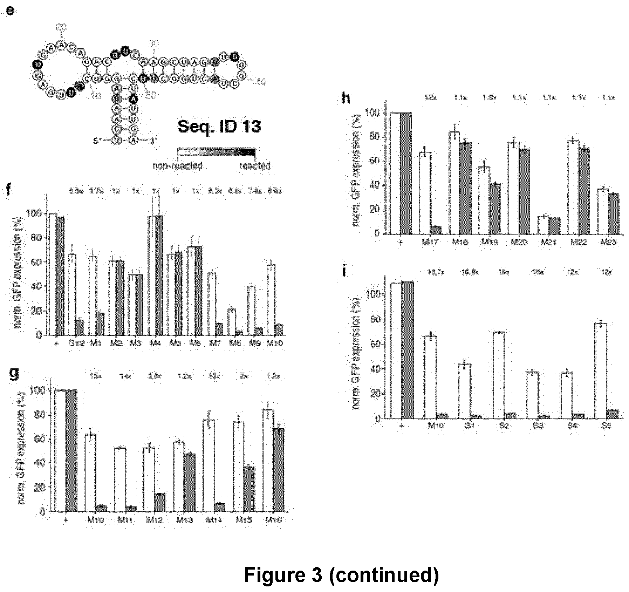

**Figure 3** - 3a) Predicted secondary structure of the full-length doxycycline-binding aptamer G12 (Seq. ID 3). Designed mutations are indicated by black boxes or grey background in the aptamer sequence and the respective mutations are named (M1-M10, Seq. ID 4-13). Direction of the RNA strand is indicated by its 5' and 3' ends. Nucleotide positions are indicated in decadic steps. 3b) Mutational studies based on the shortened M10 version of the doxycycline-binding aptamer. The mutations are indicated by black boxes or grey background in the aptamer sequence and the respective mutations are named (M11-M23, Seq. ID 14-26). Also, the stem variants for the gene regulation in the roadblock-mechanism in yeast are shown (S1-S5). Positions found to tolerate nucleotide exchange in the single nucleotide mutation screening are indicated by black filling. Direction of the RNA strand is indicated by its 5' and 3' ends. Nucleotide positions are indicated in decadic steps. 3c) Single nucleotide mutation screening. The x-axis names the position and nucleotide of the partially randomized part of M10 (Seq. ID 13) while the y-axis plots the number of reads fund for functional (white background, grey bars) or loss of function mutations (grey background, black bars). Most point mutations led to a loss of aptamer function, except for the positions 17U, 27U, 28C and 46G, as indicated by a higher number of reads accounted to functional aptamers. 3d) Single-nucleotide reactivity of the M10 (Seq. ID 13) variant in the absence of doxycycline measured with SHAPE-MaP. Reactivity of the individual positions is indicated by colouring (highest reactivity = black, lowest reactivity = white). Low reactivity of a position correlates with restricted flexibility of the nucleotide which is caused by involvement within an intramolecular structure of the aptamer. The positions of highest reactivity are 11A, 12U, 17U, 26G, 27U, 38G and 36G. 3e) Single-nucleotide reactivity of the M10 (Seq. ID 13) variant in the presence of doxycycline measured with SHAPE-MaP. Reactivity of the individual positions is indicated by colouring (highest reactivity = black, lowest reactivity = white). Low reactivity of a position correlates with restricted flexibility of the nucleotide which is caused by involvement within an intramolecular structure of the aptamer. The positions of highest reactivity are 12U, 17U, 26G, 27U, 38G and 53A. 3f) GFP expression of mutations of G12 (Seq. ID 3) measured in yeast. The sequence of the mutants is indicated in figure 3a. The GFP expression is shown in % normalized to the fluorescence of constitutive expressed mCherry and the positive control and measured in the absence and presence of 100 µM doxycycline. The "switching factor" of the individual variant is indicated above the respective columns. Mutations M2 (Seq. ID 5), M3 (Seq. ID 6), M4 (Seq. ID 7), M5 (Seq. ID 8) and M6 (Seq. ID 9) show a complete loss of regulatory function. Mutations M9 (Seq. ID 12) and M10 (Seq. ID 13) show increased switching but a reduced level of expression without doxycycline compared to G12. 3g) GFP expression of mutations of M10 (Seq. ID 13) measured in yeast. The sequence of the mutants is indicated in figure 3b. The GFP expression is shown in % normalized to the fluorescence of constitutive expressed mCherry and the positive control and measured in the absence and presence of 100 µM doxycycline. The "switching factor" of the individual variant is indicated above the respective columns. Mutations M11 (Seq. ID 14) and M14 (Seq. ID 17) maintained comparable regulatory function to M10 while all other mutations led to a decrease in regulation. 3h) GFP expression of mutations of M10 (Seq. ID 13) measured in yeast. The sequence of the mutants is indicated in figure 3b. The GFP expression is shown in % normalized to the fluorescence of constitutive expressed mCherry and the positive control and measured in the absence and presence of 100 µM doxycycline. The "switching factor" of the individual variant is indicated above the respective columns. Beside mutation M17 (Seq. ID 20), with similar regulatory behaviour to M10, all mutations led to loss of regulation. 3i) GFP expression of stem variants of M10 (Seq. ID 13) measured in yeast. The GFP expression is shown in % normalized to the fluorescence of constitutive expressed mCherry and the positive control and measured in the absence and presence of 100 µM doxycycline. The "switching factor" of the individual variant is indicated above the respective columns. Variants S1 and S2 showed improved switching behaviour compared to M10.

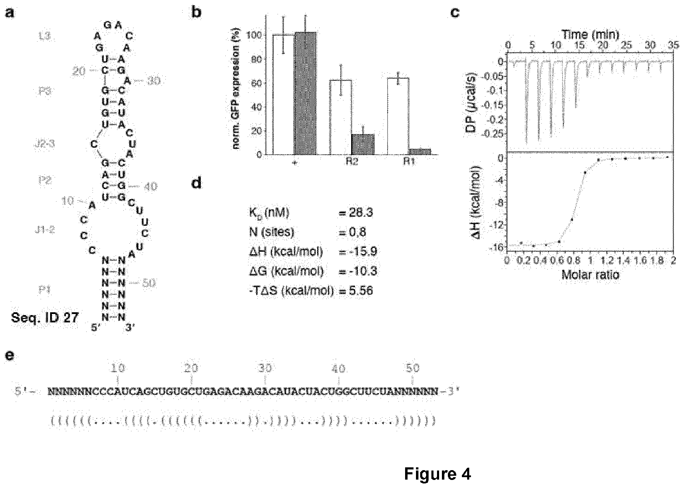

**Figure 4** - 4a) Predicted secondary of the doxycycline-binding aptamer C01 (Seq. ID 27). Direction of the RNA strand is indicated by its 5' and 3' ends. Nucleotide positions are indicated in decadic steps. 4b) GFP expression measurement of C01 (Seq. ID 27) and G12 (Seq. ID 3) in yeast. The GFP expression is shown in % normalized to the fluorescence of constitutive expressed mCherry and the positive control and measured in the absence and presence of 100 µM doxycycline. While the GFP expression in the absence of doxycycline is comparable, G12 shows a clearly lower expression level in the presence of doxycycline. 4c) Isothermal calorimetry titration (ITC) measurement of C01 binding to doxycycline for K_{D} determination with thermogram and titration curve. 4d) Resulting parameters of the binding of C01 to doxycycline resulting from the ITC measurement (4c). 4e) Generalized sequence and structure in dot-bracket annotation of C01. The basal P1 stem can be freely exchange and is therefore represented in paired "N's".

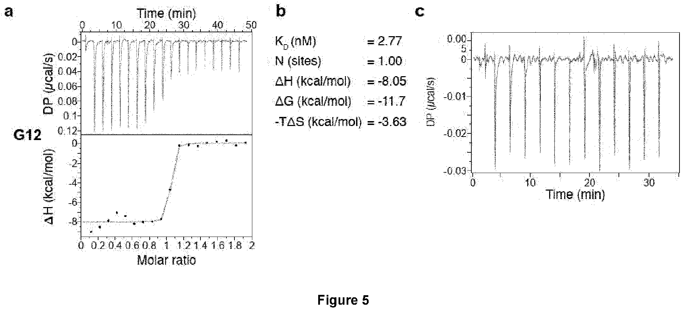

**Figure 5** - 5a) Isothermal calorimetry titration (ITC) measurement of G12 binding to doxycycline for K_{D} determination with thermogram and titration curve. 5b) Resulting parameters of the binding of C01 to doxycycline resulting from the ITC measurement (5a). 5c) ITC titration curve of tetracycline being titrated onto the G12 aptamer, showing no indication of binding events.

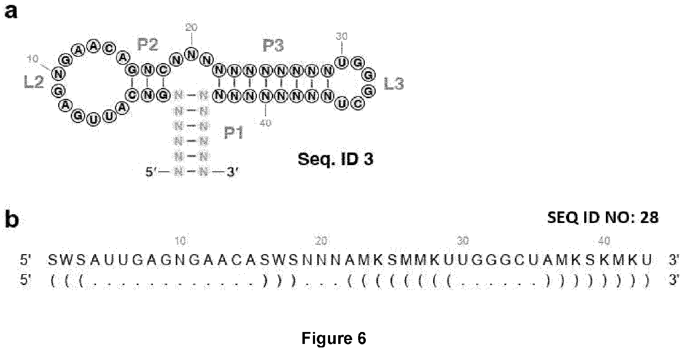

**Figure 6** - A more generalized form of the G12-Aptamer based on nucleotide-exchange-studies. 6a) Depiction of the predicted 2D structure of the aptamer according to SEQ ID NO. 3; 6b) Depiction of the further generalized aptamer sequence (SEQ ID NO: 28; nomenclature according to UIPAC-standards) with corresponding dot-bracket annotation of the predicted 2D structure.

### Detailed Description of the Disclosure

The nucleic acid molecule(s) disclosed herein are aptamers that bind with high specificity to doxycycline and undergo a conformational change upon doxycycline binding. This conformational change, which involves a transition between two distinct conformations upon doxycycline binding, is referred to herein as the "switching mechanism." This switching mechanism enables a variety of applications for the nucleic acid molecules as disclosed herein.

In one embodiment, the disclosure pertains to the nucleic acid molecule as described herein, which facilitates the regulation of gene expression in response to doxycycline binding. In another embodiment, the nucleic acid molecule disclosed herein is part of an aptazyme. In yet another embodiment, the nucleic acid molecule is part of a riboswitch.

One embodiment relates to the use of the nucleic acid molecule disclosed herein in gene regulation and/or synthetic biology. Other embodiments describe the use of the nucleic acid molecule in biosensors designed to detect doxycycline in various environments, such as clinical diagnostics or environmental monitoring. The high specificity and sensitivity of these molecules make them ideal candidates for the development of highly selective diagnostic tools and therapeutic agents.

In one embodiment the nucleic acid molecule(s) disclosed herein are characterized by an unexpectedly high switching factor, referring to the efficiency with which the molecule transitions between two functional states upon doxycycline binding. Specifically, this includes an active state in which, for example, gene expression is facilitated, and an inactive state in which gene expression is inhibited.

In further embodiments, the nucleic acid molecule(s) disclosed herein are also characterized by their high affinity for doxycycline, with a dissociation constant (Kd) of at least 3 nM, at least 4 nM, or of at least 5 nM. In one embodiment, the nucleic acid molecule(s) disclosed herein are characterized by an affinity for doxycycline of 25 nM or less, 24 nM or less, 23 nM or less. In one embodiment, the nucleic acid molecule(s) disclosed herein are characterized by an affinity for doxycycline of between 3 nM and 7 nM, between 4 nM and 6 nM, or about 5 nM ± 0.5 nM.

In case of the "C01-Mutant" the, the nucleic acid molecule(s) disclosed herein are characterized by their high affinity for doxycycline, with a dissociation constant (Kd) of at least 13 nM, at least 14 nM, or of at least 15 nM. In one embodiment, the nucleic acid molecule(s) disclosed herein are characterized by an affinity for doxycycline of 25 nM or less, 24 nM or less, 23 nM or less. In one embodiment, the nucleic acid molecule(s) disclosed herein are characterized by an affinity for doxycycline of between 13 nM and 25 nM, between 18 nM and 23 nM, or about 22 nM ± 1 nM.

In further embodiments, the nucleic acid molecule(s) disclosed herein are characterized by their high specificity for doxycycline. In this context, "high specificity" means that the molecule binds doxycycline with strong affinity (a Kd of at least 3 nM, 4 nM, or 5 nM) but does not bind to the structurally related tetracycline (as measured by isothermal Titration Caliometry (ITC)).

### G12-Mutant

In one embodiment the disclosure relates to a nucleic acid molecule comprising the general structure of:
5'-GNCAUUGAGNGAACAGNCNNNNNNNNNNNUGGGCUNNNNNNNN-3'(SEQ ID NO: 1)
wherein "N" can be replaced by any nucleotide selected from the group consisting of Thymine (T), Uracil (U), Cytosine (C), Adenine (A), and Guanine (G).

The general structure can also be represented in a "dot-bracket"-notation:
5'-GUCAUUGAGNGAACAGNCNNNNNNNNNNNUGGGCUNNNNNNNN-3' (SEQ ID NO: 1)
5'-(((............)))...((((((((......))))))))-3'
wherein the dot-bracket notation represents the RNAs secondary structure. In this system dots (.) represent unpaired nucleotides, opening brackets ((and closing brackets)) represent base pairs (e.g., hydrogen bonds between complementary nucleotides). For example, ((..)) indicates that two nucleotides at the beginning and end of the sequence form a base pair, while the two nucleotides in the middle remain unpaired, please also see the structural depiction in figure 1a and 1b.

The skilled person knows that nucleotides which need to pair, and are represented by opening and closing brackets, can only be exchanged with their respective complementary counterpart, i.e. nucleic acid base pairing follows specific rules in both RNA and DNA, based on complementary hydrogen bonding. In DNA, the four nitrogenous bases-adenine (A), thymine (T), cytosine (C), and guanine (G)-pair in a specific manner: adenine forms two hydrogen bonds with thymine, while cytosine forms three hydrogen bonds with guanine. This pairing ensures the stability of the DNA double helix. In RNA, thymine is replaced by uracil (U), meaning that adenine pairs with uracil instead of thymine, while cytosine still pairs with guanine. In contrast, nucleotides depicted as non-pairing allow more freedom to choose for variations.

In one embodiment, also artificial nucleotides may be used. Several artificial nucleotides have been developed to simulate base pairing in nucleic acid molecules, including riboswitches. Unnatural base pairs (UBPs) such as d5SICS and dNaM form a stable hydrophobic base pair without hydrogen bonding, enabling replication and transcription in modified DNA and RNA systems. Other synthetic bases, such as PICS (pyridone-based bases), mimic natural stacking interactions while expanding the range of possible base pairs. Hachimoji DNA and RNA introduce four additional bases-Z, P, S, and B-which maintain Watson-Crick-like pairing while expanding the genetic alphabet, offering new possibilities for synthetic biology and artificial riboswitch design. Fluorophore-modified bases, such as 2-aminopurine or pyrene derivatives, allow for real-time monitoring of riboswitch conformational changes in response to ligand binding. Additionally, locked nucleic acids (LNAs) and peptide nucleic acids (PNAs) enhance structural stability and specificity, making them valuable for engineering functional riboswitches with improved properties. These artificial nucleotides provide powerful tools for designing novel genetic elements with enhanced sensitivity, specificity, or entirely new functions.

A generation of several mutations of the original sequence and function studies revealed a number of mutations, which are of particular interest. Thus, in one embodiment the disclosure relates to a nucleic acid molecule comprising the general structure of (SEQ ID NO: 29):

10 20 30 40
5' SWSAUUGAGNGAACASWSNNNAMKSMMKUUGGGCUAMKSKMKU 3'
5' (((............)))...((((((((......)))))))) 3'

Wherein the one-letter code follows the denomination of IUPAC-Nomenclature Committee of the International Union of Biochemistry (NC-IUB) (1984). "Nomenclature for Incompletely Specified Bases in Nucleic Acid Sequences". Nucleic Acids Research. 13 (9): 3021-3030. doi:10.1093/nar/13.9.3021. PMC 341218. PMID 2582368;
wherein "N" can be replaced by any nucleotide selected from the group consisting of Thymine (T), Uracil (U), Cytosine (C), Adenine (A), and Guanine (G), or any of the artificial counterparts as disclosed hereinunder;
wherein "K" is a ketone-based nucleotide, such as Thymine (T) or Guanine (G); "M" is a amino-based nucleotide, such as Adenine (A) or Cytosine (C);
wherein "W" is a weak interaction, such as Adenine (A) and Uracil (U) or Thymine (T); "S" is a strong interaction, such as Cytosine (C) and Guanine (G);
wherein "R" is a pyrimidine; "Y" is a purine; "D" is not Guanine (G); "B" is not Thymine (T) or Uracil (U); "V" is not Adenine (A); "H" is not Cytosine (C).

In one embodiment the disclosure relates to a nucleic acid molecule selected from the group of sequences of SEQ ID NO: 3, 4, 10, 11, 12, 13, 14, 15, 17, 18, and 20 (cf. also table 1).

In one embodiment the disclosure relates to a nucleic acid molecule selected from the group of sequences of SEQ ID NO: 3, 4, 10, 11, 12, 13, 14, 17, and 20 (cf. also table 1).

Further, in a loss-of-function study it has been found that the flexibility of exchange of the different nucleotides depends on the position of the nucleotide within the molecule (cf. figure 1c). That is that mainly nucleotides in position 11, 20-22, and 40 can be exchanged by any nucleotide selected from the group consisting of Thymine (T), Uracil (U), Cytosine (C), Adenine (A), and Guanine (G) without loss of function, whereas exchanges at other positions may to some degree influence the functionality of the molecule.

Thus, in one embodiment the disclosure relates to a nucleic acid molecule comprising the general structure of
5'-GGUCAUUGAGNGAACAGACNNNAAGCUAGUUGGGCUACUNGCUUC-3' (SEQ ID NO: 2) wherein "N" can be replaced by a nucleotide selected from the group consisting of Thymine (T), Uracil (U), Cytosine (C), Adenine (A), and Guanine (G) or any of the artificial counterparts as disclosed hereinunder.

In one embodiment the disclosure relates to a nucleic acid molecule comprising the sequence of at least one of the sequences selected from the group of SEQ ID NO: 1-27 & 29, as well as sequences with a sequence identity of at least 90%, at least 95%, at least 98%, at least 99%, or at least 99,9%.

### C01-Mutant

In one embodiment the disclosure relates to a nucleic acid molecule comprising the general sequence of:
5'-NNNNNNCCCAUCAGCUGUGCUGAGACAAGACAUACUACUGGCUUCUANNNNNN-3' (SEQ ID NO: 27)
**wherein** "N" can be replaced by any nucleotide selected from the group consisting of Thymine (T), Uracil (U), Cytosine (C), Adenine (A), and Guanine (G).

The general structure can also be represented in a "dot-bracket"-notation:
5'-NNNNNNCCCAUCAGCUGUGCUGAGACAAGACAUACUACUGGCUUCUANNNNNN-3'
5'-((((((....((((.((((((......)).))))...))))......))))))-3'
wherein the dot-bracket notation represents the RNAs secondary structure.

In one embodiment the disclosure relates to a nucleic acid molecule comprising the sequence of SEQ ID NO: 28.

In one embodiment the disclosure relates to a nucleic acid molecule comprising the sequence of at least one of the sequences selected from the group of SEQ ID NO: 27 and 28, as well as sequences with a sequence identity of at least 90%, at least 95%, at least 98%, at least 99%, or at least 99,9%.

In one embodiment the disclosed nucleic acid molecules do have a switching factor of, at least 2, at least 2.25, at least 2.5, at least 2.75, at least 3, at least 5, at least 7.5, at least 10, at least 15, at least 20, or at least 25, wherein the switching factor is measured based on GFP-(green fluorescent protein)-expression, i.e. reduction of GFP expression upon addition of doxycycline. For example, a switching factor of 5 therefore means the reduction of GFP expression upon addition of doxycycline by a factor of 5. In some embodiments the switching factor is up to 25, up to 20, up to 15, up to 10, up to 7.5, up to 5, or up to 3. In some embodiments the switching factor is from 2 to 25, from 2.25 to 20, from 2.5 to 15, from 3 to 12, or from 3.25 to 10.

In one embodiment the disclosed nucleic acid molecules bind to doxycycline with an affinity (Kd) of 20 nM or less, of 15 nM or less, 10 nM or less, 5 nM or less, or 2 nM or less. In one embodiment the disclosed nucleic acid molecules bind to doxycycline with an affinity (Kd) of at least 2 nM, of at least nM, of at least nM, of at least nM, or at least nM. In one embodiment the disclosed nucleic acid molecules bind to doxycycline with an affinity (Kd) from 2 nM to 20 nM, from 5 nM to 18 nM, or from 10 nM to 15 nM. In one embodiment the binding occurs with no binding to tetracycline detectable by Isothermal Titration Calorimetry (ITC).

In one embodiment the disclosed nucleic acid molecules bind to doxycycline with a limit-of-detection (LOD) of less than 5 nM, of less than 3 nM, of less than 2 nM, or of less than 1 nM. In one embodiment the disclosed nucleic acid molecules bind to doxycycline with a limit-of-detection (LOD) of at least 1 nM, of less than 2 nM, of less than 3 nM, or of less than 5 nM. In one embodiment the disclosed nucleic acid molecules bind to doxycycline with a limit-of-detection (LOD) from 1 nM to 5 nM, from 1 nM to 3 nM, or from 1 nM to 2 nM.

In one embodiment the binding of the nucleic acid molecules to doxycycline is measured by Isothermal Titration Calorimetry (ITC). Isothermal Titration Calorimetry (ITC) is a biophysical technique used to measure the thermodynamics of molecular interactions in solution. ITC directly determines the heat changes associated with the binding of two molecules, providing detailed information about the binding process.

In general, for ITC a ligand or small molecule is titrated into a sample containing the binding partner (e.g., a protein) in an isothermal environment. The binding reaction releases or absorbs heat, depending on whether the interaction is exothermic (releases heat) or endothermic (absorbs heat). This heat change is measured in real time, and the resulting data can be used to calculate:
- Binding affinity (K_{d})
- Enthalpy change (ΔH)
- Entropy change (ΔS)
- Stoichiometry of the interaction (binding ratio).

In one embodiment the binding of the nucleic acid molecules disclosed hereinunder undergoes a conformational change upon doxycycline-binding. In one embodiment this conformational change results in a gene-regulation selected from the group consisting of inhibition of transcription of a gene, activation of transcription of a gene, regulation of translation of a gene, regulating ribozyme activity, regulating nucleic acid hybridization, or affection of splicing of a gene, as well as combinations thereof.

In one embodiment the disclosure relates to the use of the disclosed nucleic acid molecules for gene-regulation. In one embodiment the disclosure relates to the use of disclosed the nucleic acid molecules for gene-regulation selected from the group consisting of inhibition of transcription of a gene, activation of transcription of a gene, regulation of translation of a gene, or affection of splicing of a gene, as well as combinations thereof.

In one embodiment the disclosure relates to the use of the disclosed nucleic acid molecules, wherein the molecules are combined with a second system for gene-regulation selected from the group comprising a Tet-On-system, CRISPR-Cas9-system, RNAi-system, ZINC Finger Nuclease (ZFN)-system, Transcription Activator-Like Effector Nucleases (TALENs)-system, further aptamer-systems, an optogenetics system, a heat-inducible system, a chemical-inducible system, and a protein dimerization system, as well as combinations thereof.

The nucleic acid molecules of the present disclosure do have a wide range of applications beyond being used as a riboswitch or an aptazyme. One potential application is in the field of bio-sensing, where the conformational change could trigger a detectable signal, such as a fluorescence or colorimetric change, indicating the presence of doxycycline. This would be useful for environmental monitoring, clinical diagnostics, or pharmaceutical quality control. Additionally, such a molecule can be used in targeted drug delivery systems, where the binding event could act as a trigger to release a therapeutic agent, enabling controlled release in response to doxycycline. Furthermore, it could be integrated into molecular switches or devices for synthetic biology, where doxycycline presence could regulate cellular processes or initiate a cascade of reactions. The conformational change could also be utilized in the creation of molecular memory systems, where the aptamer "remembers" the presence of doxycycline for extended periods, adding to the potential applications in biotechnology and bioengineering.

In one embodiment the disclosure relates to the use of the disclosed nucleic acid molecules as part of a riboswitch, as part of an aptazyme, biosensor, targeted drug delivery system, molecular switches or devices for synthetic biology, or molecular memory systems.

In one embodiment the disclosure relates to the use of the disclosed nucleic acid molecules for gene-regulation selected from the group consisting of inhibition of transcription of a gene, activation of transcription of a gene, regulation of translation of a gene, or affection of splicing of a gene, as well as combinations thereof.

In one embodiment the disclosure relates to the use of the disclosed nucleic acid molecules wherein the molecule is combined with a second system for gene-regulation selected from the group comprising a Tet-On-system, CRISPR-Cas9-system, RNAi-system, ZINC Finger Nuclease (ZFN)-system, Transcription Activator-Like Effector Nucleases (TALENs)-system, further aptamer-systems, an optogenetics system, a heat-inducible system, a chemical-inducible system, and a protein dimerization system, as well as combinations thereof.

In further embodiments the disclosure relates to pharmaceutical compositions comprising the nucleic acid molecules as disclosed.

In one embodiment, the pharmaceutical formulation comprises at least one type of vectors or carriers selected from the group of viral vectors (for example adenoviral vectors, lentiviral vectors, and/or adeno-associated viral (AAV) vectors), non-viral vectors, such as liposomes, polymeric nanoparticles, cationic lipids or polymers, and/or exosomes.

In further embodiments the pharmaceutical formulation comprises at least one component selected from the group consisting of fillers, binders, disintegrants, lubricants, glidants, buffering agents, preservatives, stabilizers, coating agents, solvents, surfactants, and humectants, as well as combinations thereof.

In further embodiments the disclosure relates to nucleic acid molecules disclosed hereinunder, or the pharmaceutical compositions disclosed hereinunder, for use as a medicament. In yet another embodiment, the disclosure relates to nucleic acid molecules disclosed hereinunder, or the pharmaceutical compositions disclosed hereinunder, for use in the treatment of a disease where precise, tunable, and reversible control of gene expression is advantageous. This includes diseases like genetic disorders, cancer, and infectious diseases.

In the case of genetic disorders, such as cystic fibrosis or Duchenne muscular dystrophy, riboswitches could regulate the expression of a therapeutic gene, allowing treatment to be tailored to the patient's needs while minimizing side effects. For cancer, doxycycline-controlled riboswitches could be used to regulate the production of tumor-suppressing proteins or to activate genes encoding pro-apoptotic factors selectively in cancerous cells. The ability to stop gene expression by withdrawing doxycycline could also serve as a safety mechanism to halt therapy if adverse effects occur. In infectious diseases, such as bacterial or viral infections, riboswitches could be applied to control the expression of antiviral or antibacterial genes in a way that minimizes toxicity and reduces the risk of resistance. For chronic conditions, such as autoimmune diseases, riboswitches could be used to regulate immunomodulatory proteins, offering precise control to prevent overactivation or suppression of the immune system.

### Sequences

The following sequences are part of this disclosure.

All Sequences are shown in DNA as encoded on the plasmid for testing. Starting at the 5' end with the terminal "U" of the basal stem of each variant and ending with the "AUG" in front of the reporter gene GFP.

**Table 1: Sequences of G12 and its tested mutations.**

| In one embodiment the constructs of the present disclosure comprise at least one of the sequences selected from the group consisting of Seq ID NO: 3 - 26 and 28, as depicted below. | | |
|---|---|---|
| **Variant** | **SEQ ID NO:** | **Sequence 5' - 3'** |
| G12 | 3 | TACCGGTAATTTAGGTCATTGAGTGAACAGACGTCAAGCTAGTTGGGCTACTGGCTT- CTAAAACACAGGTAAAATG |
| M1 | 4 | TACCGGTAATTTAGGTCATTGAGTGAACAGACGTCAAGCAGTTGGGCTACTGCTT- CTAAAACACAGGTAAAATG |
| M2 | 5 | TACCGGTAATTTAGGTCATTGAGTGAACAGACGTCAGCAGTT- GGGCTACTGCTCTAAAACACAGGTAAAATG |
| M3 | 6 | TACCGGTAATTTAGGTCATTGAGTGAACAGACGTCAGCGTT- GGGCTACGCTCTAAAACACAGGTAAAATG |
| M4 | 7 | TACCGGTAATTTAGGTCATTGAGTGAACAGACGTCAAGCTAGTAAAGACTGGCTT- CTAAAACACAGGTAAAATG |
| M5 | 8 | TACCGGTAATTTAGGTCATTGAGTGAACAGACGTCAAGCTAGTTTCGACTGGCTT- CTAAAACACAGGTAAAATG |
| M6 | 9 | TACCGGTAATTTAGGTCATTGAGTGAACAGACGTCAACGTAGTTGGGCTACTGGCTT- CTAAAACACAGGTAAAATG |
| M7 | 10 | TACCGGTAATTTAGGTCATTGAGTGAACAGACGTCAACGTAGTTGGGCTACTGGCTT- CTAAAACACAGGTAAAATG |
| M8 | 11 | TACCGGTAATTTAGGTCATTGAGTGAACAGACGTCAAGCTAGTTGGGCTACTGGCTT- CTAAAACACCGGTAAAATG |
| M9 | 12 | TACCGGTAATTTAGGTCATTGAGTGAACAGACGTCAAGCTAGTTGGGCTACTGGCTT- CTAAATTACAGGTAAAATG |
| M10 | 13 | TACCGGTCAATAGGTCATTGAGTGAACAGACGTCAAGCTAGTTGGGCTACTGGCTTCTATT- GAAAAATG |
| M11 | 14 | TACCGGTCAATAGGTCATTGAGTGAACAGACGTCAGACCGATTGGGCTATCGGTCTCTATT- GAAAAATG |
| M12 | 15 | TACCGGTCAATAGGTCATTGAGTGAACAGACGTCAGACCGATTGGGCTATCGGTCTCTATT- GAAAAATG |
| M13 | 16 | TACCGGTCAATAGGTCATTGAGTGAACAGACGTCAAGCTACAGTTGGGCTACTGTGGCTT- CTATTGAAAAATG |
| M14 | 17 | TACCGGTCAATAGGTCATTGAGTGAACAGACAAAAAGCTAGTTGGGCTACTGGCTTCTATT- GAAAAATG |
| M15 | 18 | TACCGGTCAATAGCTGATTGAGTGAACACAGGTCAAGCTAGTTGGGCTACTGGCTTCTATT- GAAAAATG |
| M16 | 19 | TACCGGTCAATAGGACATTGAGTGAACAGACGTCAAGCTAGTTGGGCTACTGGCTTCTATT- GAAAAATG |
| M17 | 20 | TACCGGTCAATAGGACATTGAGTGAACAGTCGTCAAGCTAGTTGGGCTACTGGCTTCTATT- GAAAAATG |
| M18 | 21 | TACCGGTCAATAGGAGATTGAGTGAACACACGTCAAGCTAGTTGGGCTACTGGCTTCTATT- GAAAAATG |
| M19 | 22 | TACCGGTCAATAGGAGATTGAGTGAACACTCGTCAAGCTAGTTGGGCTACTGGCTTCTATT- GAAAAATG |
| M20 | 23 | TACCGGTCAATAGGTCCAAGAGTGAACAGACGTCAAGCTAGTTGGGCTACTGGCTTCTATT- GAAAAATG |
| M21 | 24 | TACCGGTCAATAGGTCATTCAATGAACAGACGTCAAGCTAGTTGGGCTACTGGCTTCTATT- GAAAAATG |
| M22 | 25 | TACCGGTCAATAGGTCATTGAGCAAACAGACGTCAAGCTAGTTGGGCTACTGGCTTCTATT- GAAAAATG |
| M23 | 26 | TACCGGTCAATAGGTCATTGAGTGACAAGACGTCAAGCTAGTTGGGCTACTGGCTTCTATT- GAAAAATG |
| C01 | 28 | AGGTTAGGCG CCCATCAGCT GTGCTGAGAC AAGACATACTACTGGCTTCTACGTTTAACCT |

The aptamers can be combined with different stem-sequences depicted in the following table. Please see also figure 3 a and b, which shows the overall structure of the different constructs.

Based on different sequence- and structure-evaluations (cf. examples and figures), generalized sequences can be predicted. Therein certain nucleic acids need to be conserved to maintain the advantageous function, whereas others can be varied to a certain extent. The skilled person can use these generalized sequences to find close related variants which also are encompassed by this disclosure.

**Table 2: Generalized variants of the constructs.**

| In one embodiment the constructs of the present disclosure comprise at least one of the sequences encompassed in the generalized sequences selected from the group consisting of Seq ID NO: 1, 2, 27 and 28, as depicted below. | | |
|---|---|---|
| **Variant** | **SEQ ID NO: NO:** | **Sequence 5' - 3'** |
| G12-variant 01 | 1 | gncattgagn gaacagacnn nnnnnnnnnt gggctnnnnn nnn |
| G12-variant 02 | 2 | ggtcattgag ngaacagacn nnaagctagt tgggctactn gcttc |
| G12-variant 03 | 28 | swsattgagn gaacaswsnn namksmmktt gggctamksk mkt |
| C01-variant | 27 | nnnnnnccca tcagctgtgc tgagacaaga catactactg gcttctannn nnn |

wherein "N" can be replaced by any nucleotide selected from the group consisting of Thymine (T), Uracil (U), Cytosine (C), Adenine (A), and Guanine (G), or any of the artificial counterparts as disclosed hereinunder;
wherein "K" is a ketone-based nucleotide, such as Thymine (T) or Guanine (G); "M" is an amino-based nucleotide, such as Adenine (A) or Cytosine (C);
wherein "W" is a weak interaction, such as Adenine (A) and Uracil (U) or Thymine (T); "S" is a strong interaction, such as Cytosine (C) and Guanine (G);
wherein "R" is a pyrimidine; "Y" is a purine; "D" is not Guanine (G); "B" is not Thymine (T) or Uracil (U); "V" is not Adenine (A); "H" is not Cytosine (C).

**Table 3: Different "stem"-sequences which were found to be beneficial for the overall construct. In one embodiment the constructs of the present disclosure comprise at least one of the sequences selected from the group consisting of P1, S1, S2, S3, S4 and S5 as depicted below.**

| **Variant** | **SEQ ID NO:** | **Sequence 5' - 3'** |
|---|---|---|
| P1 | Too short for SEQ ID NO: | UCAACAG-[Aptamer]-CUAUUGA |
| S1 | Too short for SEQ ID NO: | UCAAGCG-[Aptamer]-CCGUUGA |
| S2 | Too short for SEQ ID NO: | UCGAUAG-[Aptamer]-CUAUCGA |
| S3 | Too short for SEQ ID NO: | UCGCUAG-[Aptamer]-CUAGCGA |
| S4 | Too short for SEQ ID NO: | UCGCUAG-[Aptamer]-CUAGUGA |
| S5 | Too short for SEQ ID NO: | UCGAUAG-[Aptamer]-CUAUUGA |

### Definitions

The term "aptazyme" is used hereinunder for engineered RNA molecules that combine the properties of an aptamer and a ribozyme. The aptamer binds to a specific target, such doxycycline, while the ribozyme catalyzes a biochemical reaction, such as RNA cleavage. The binding of the target induces a conformational change in the RNA, activating or inhibiting the ribozyme's catalytic function. Aptazymes are used in applications like biosensors, gene regulation, and synthetic biology, allowing for programmable RNA circuits that respond to specific signals.

The term "gene-regulatory sequence" of a riboswitch refers to a specific segment of RNA that regulates the expression of a gene in response to binding with a small molecule, in the present disclosure doxycycline. Riboswitches are non-coding RNA elements typically found in the 5' untranslated region (UTR) of mRNA. When the small molecule binds to the riboswitch, it induces a conformational change in the RNA structure, which can affect gene expression by influencing processes such as transcription, translation, or stability of the mRNA. In essence, the gene-regulatory sequence within the riboswitch controls gene expression by responding to environmental or cellular cues, often through a mechanism involving the alteration of the RNA's structure that either blocks or promotes the translation of the downstream gene. This allows cells to adapt to changing conditions in an efficient and regulatory manner.

The term "gene regulation" as used hereinunder, means "gene regulation mediated by a riboswitch" and refers to the control of gene expression through the binding of specific metabolites or small molecules to an RNA element, typically located in the untranslated region (UTR) of an mRNA transcript. This binding induces conformational changes in the riboswitch, which can either promote or inhibit the expression of the gene. The riboswitch acts as a regulatory "switch" by altering the structure of the RNA, affecting processes like transcription or translation. In many cases, riboswitches regulate gene expression by controlling the access of ribosomes to the ribosome-binding site (RBS) or by influencing transcription termination. Through this mechanism, riboswitches enable cells to rapidly adjust gene expression in response to fluctuating levels of metabolites, playing an essential role in metabolic control.

The term "switching factor" is used hereinunder to refer to the effectiveness with which a riboswitch transitions between its two functional states: an active state, wherein gene expression is facilitated, and an inactive state, wherein gene expression is inhibited. The switching factor typically represents the ratio of gene expression levels in the absence and presence of the specific ligand that activates or represses the riboswitch.

A high switching factor indicates that the riboswitch exhibits a pronounced difference in gene expression between these states, ensuring precise and robust regulation. This parameter encompasses aspects such as:
- Dynamic range: The magnitude of the difference in gene expression levels between the active and inactive states.
- Sensitivity: The responsiveness of the riboswitch to ligand concentrations.
- Cooperativity: The involvement of multiple ligand-binding sites in modulating the switching behavior.

The switching factor can be experimentally determined, for example, by measuring reporter gene expression in a system containing the riboswitch under defined ligand conditions.

For example, a "switching factor of 25" means that the level of gene expression in the active state (e.g., when the ligand is bound to the riboswitch) is 25 times higher than in the inactive state (e.g., when the ligand is absent).

If, for instance, the gene expression level in the inactive state is measured at 10 arbitrary units (AU), then in the active state, the expression would rise to 250 AU. This demonstrates the riboswitch's ability to strongly regulate gene expression depending on the presence or absence of its ligand.

For simplicity, some sequences are shown using only thymine (T) or uracil (U); however, these nucleotides can be used interchangeably depending on the specific context. In DNA, thymine (T) is preferred, while in RNA, uracil (U) is the standard base.

Nomenclature used in this disclosure:
- "G12" - doxycycline-binding aptamer according to SEQ ID NO: 1-3, or SEQ ID NO: 29.
- "M1-M23" - Mutants of G12 - doxycycline-binding aptamer (SEQ ID NO: Nos. 4-26).
- "C01" - doxycycline-binding aptamer according to SEQ ID NO: 27-28.

### Examples

### Example 1: Discovery of doxycycline-binding aptamers with regulatory potential by parallel in vitro selection and subsequent in vivo screening

The in vitro selection for a new doxycycline-binding aptamer was performed in parallel using Capture-SELEX and column-based SELEX. Selection was started with 1,2 x 10¹⁶ individual molecules of the Capture-SELEX pool performing column-based SELEX. Within 5 rounds of selection using 500 µM doxycycline for elution, the pool showed first signs of binding which further increased in round S6. From round S7 to S9, a counter-elution step with 500 µM kanamycin B as a counter-elution step was introduced to remove weak binding candidates from the pool. Counter-elution led to an expected decrease in the eluted fraction with doxycycline but recovered until round S9. For round S10, the counter-elution was left out and selection stringency was increased by reducing the doxycycline concentration in the elution to 50 µM. This round resulted in the highest eluted fraction with 35%, implying a clear enrichment of doxycycline-binding candidates. Capture-SELEX was performed as previously described with the same Capture-SELEX pool as used for the column-based SELEX and 1 mM of doxycycline for the elution of candidates. In five attempts no elution of RNA was observed over up to 10 rounds of selection. In response, the doxycycline concentration used was reduced to 100 µM and after six rounds of selection an increased elution of RNA was observed and further increased in round C7. Repeating the selection round C7 with a concentration range from 100 µM to 1 mM confirmed a negative effect of doxycycline concentrations over 600 µM on the elution of RNA in the Capture-SELEX. With eluted fractions of 35% and 37% in column-based and Capture-SELEX, respectively, the RNA pools of both selections showed clear signs of enrichment for target binding and were subsequently used for in vivo screening in yeast.

To identify aptamers suited for riboswitch engineering, pools of round S6, S8 and S10 from the column-based SELEX and of round C5 and C7 from the Capture-SELEX were used for an in vivo screening in *Saccharomyces cerevisiae.* The DNA pool of the respective rounds was inserted by homologous recombination in the 5'UTR upstream of the coding sequence of GFP, to function as a roadblock upon binding to their ligand through blocking ribosomal scanning as previously described. Based on the roadblock mechanism, aptamers with regulatory potential should allow for GFP expression through a rather loose structure, while reducing GFP expression upon addition of doxycycline by an increase in stability. To remove candidates with too high stability, unsuited for riboswitch engineering, the pools were pre-selected by FACS for GFP expression in the absence of doxycycline. With progressing rounds, the fraction of suited candidates decreased in the column-based SELEX, leaving 13% suited for screening in round S10.

This was not observed for the pools originating from the Capture-SELEX. Among the pre-selected candidates from the column-based SELEX no noticeable switching behavior was observed upon addition of doxycycline, except for a single candidate with a 7x switching factor. In contrast, the pool from the Capture-SELEX showed a mean switching factor of around 3x for round C5 and C7 (fig. 1e), indicating a great fraction of candidates with prominent switching behavior in these pools. The clones possessing regulatory potential were individually remeasured to confirm the switching behavior before sequencing. This revealed a single aptamer with regulatory potential from the column-based SELEX pool of round 10 with a switching factor of 5,5-fold and a basal expression level of 65% named "G12" when remeasured. In contrast, the fraction with regulatory potential in the Capture-SELEX rounds consisted of a single candidate with a remeasured switching factor of 3-fold and a basal expression level of 65% named "C01".

### Example 2: Characterization and optimization of the new doxycycline-binding aptamer G12

To further assess the potential of both aptamers for the engineering of riboswitches, binding affinity to doxycycline was measured using isothermal titration calorimetry (ITC), revealing KD values of 2.8 nM for G12 and 28 nM for C01 (fig. 5). To confirm the results of the ITC measurements, fluorescence titration with doxycycline was performed. For G12 fluorescence titration measurements resulted in a KD of 4,8 ± 0,1 nM, confirming the ITC results. In contrast, no fluorescence was detected for the binding of C01 to doxycycline, indicating a different binding mechanism of C01 compared to G12. For both aptamers no binding to tetracycline was observed by ITC nor fluorescence titration. Based on the greater regulatory potential and higher affinity to doxycycline, G12 was chosen for further characterization and optimization.

First, the structure of G12 was tested by mutations and truncations to reduce size and gain insights into the potential binding site of doxycycline. As a starting point for the design of mutations, a 2D folding prediction was created using the ViennaRNA package (University of Vienna, fig. 3a & 3b). Rational mutations were designed, targeting all important structural sections of the aptamer. While measuring G12 as a reference to the mutations, newly transformed yeast containing G12 on the original plasmid revealed a consistent, increased switching factor of 10-fold. Regulatory performance of the mutations suggested that the P1 and P3 stem mainly serve structural function. For the P3 stem it was shown that a length of 8 base pairs led to optimal aptamer functionality, decreasing switching capability when changing the length of the stem like in mutants M1, M2, M3, M12 and M13 while maintaining functionality when the sequence was inverted as shown by mutant M11. For the P1 stem the best combination of switching factor and basal expression was achieved with a length of 7 base pairs in form of mutant M10. In contrast, in the P2 stem only mutations of the middle base pair were tolerated, greatly losing switching behavior when mutating the border base pairs as shown by mutants M15, M17 and M19. Exchanging the central junction in mutant M14 showed complete exchangeability without losing aptamer function. Mutations in the L2 or L3 region led to complete loss of switching behavior as demonstrated by mutants M4, M5, M20, M21, M22 and M23, indicating the important contribution of these sections to ligand binding and tertiary conformation. To further assess mutation effects on a single nucleotide level, partly randomized sequences of the shortened aptamer M10 were brought into yeast, resulting in a mutation rate of 3% per nucleotide from position 7 to 51. The mutated pool of M10 was then screened in yeast for GFP expression in the presence or absence of doxycycline and sorted based on the threshold performance values of M10. Mutants showing decreased or loss of switching behavior were accounted as loss of functions mutants, while mutants maintaining the behavior of M10 were accounted as functional mutants. Results supported the findings of the rational mutations, with mutation toleration in the J1-3 junction region, a tolerated G to A mutation in the P3 stem maintaining its predicted structure and no mutation tolerance in the L2 and L3 region, except for the position U17 (fig. 3b & 3c).

To further improve gene regulation in yeast, M10 was used as a basis to create the stem variants S1-S5. As previously reported, the stability of the P1 stem greatly influences the switching behavior in yeast when used as a roadblock. Increased stability was achieved by exchanging A-U base pairs against G-C pairs. Also, G∘U pairs were inserted to increase flexibility in the unbound state. Mutations were designed to achieve a maximal range of stability between ligand-bound and -unbound state to increase the switching factor. Overall, the change of A-U pairs to G-C pairs reduced the expression levels in absence and presence of doxycycline, thereby affecting the switching behavior. The tested insertions of G∘U pairs in contrast mainly increased the expression levels in the presence of doxycycline, thereby decreasing the switching factor. From these stem variants, the biggest improvement in switching behavior was observed for S1 which was created by exchanging two A-U pairs for two G-C pairs. This variant possessed a 20-fold switching behavior upon addition of 100 µM doxycycline with a basal expression level of 40% (fig. 3i). To test whether the improvement in switching capability of the P1 stem variants M10 and S1 compared to G12 was caused by improved binding of doxycycline or a changed structural switching behavior, the K_{D} of M10 and S1 was measured by ITC and fluorescence titration. Equal affinities to doxycycline measured for the stem variants and G12 indicate that the changes in the P1 stem do not affect ligand binding. To further test if the changes in the P1 stem altered the behavior of the aptamer, the aptamers were tested in yeast with a range of doxycycline concentrations and the EC50 was calculated. All three versions of the aptamer possessed a EC50 around 2,7 µM (fig. 2b), further confirming identical ligand-aptamer interaction behavior.

Overall, these mutational studies lead to the conclusion that the most important parts for the aptamer to function are the L2 and L3 region with almost no variability, while the P2 and especially the P3 region seemed to be of structural nature. Also, the P1 stem serves merely structural function and can be adapted in its structure and sequence for any application of the aptamer.

Measurements were performed in yeast utilizing a "roadblock mechanism" for translation control by placing the aptamers in the 5'UTR of a GFP mRNA. The expression was measured without doxycycline ("ON") and with the addition of 100 µM doxycycline("OFF"). Based on the structural dynamic of the tested candidate, the translation should occur without ligand and be inhibited upon ligand addition. The ON and OFF expression levels are shown normalized in % relative to a positive control without any aptamer structure in the 5'UTR.

**Table 4: Overview of regulatory abilities of G12 mutations.**

| Variant | **ON** (%) | **OFF** (%) | **SF** (x-fold) |
|---|---|---|---|
| **G12** | 69,8 | 6,4 | **10,9** |
| **M1** | 66,4 | 9,7 | **6,8** |
| **M2** | 61,3 | 58,5 | **1,0** |
| **M3** | 49,3 | 46,8 | **1,1** |
| **M4** | 92,6 | 90,6 | **1,0** |
| **M5** | 66,8 | 66,6 | **1,0** |
| **M6** | 75 | 68,4 | **1,1** |
| **M7** | 47,7 | 4,9 | **9,7** |
| **M8** | 21,2 | 2,3 | **9,2** |
| **M9** | 44,4 | 3,2 | **13,9** |
| **M10** | 65,7 | 4,2 | **15,6** |
| **M11** | 52,8 | 3,6 | **14,7** |
| **M12** | 52,6 | 14,8 | **3,6** |
| **M13** | 57,4 | 48 | **1,2** |
| **M14** | 76 | 6 | **12,7** |
| **M15** | 73,5 | 37,3 | **2,0** |
| **M16** | 82,9 | 68,9 | **1,2** |
| **M17** | 66,4 | 5,6 | **11,9** |
| **M18** | 84,1 | 75,4 | **1,1** |
| **M19** | 54,5 | 41,6 | **1,3** |
| **M20** | 75,3 | 70,2 | **1,1** |
| **M21** | 14,8 | 13,2 | **1,1** |
| **M22** | 77 | 70,8 | **1,1** |
| **M23** | 37,1 | 33,44 | **1,1** |

### Example 3: In order to assess the regulatory potential of the novel doxycycline-binding aptamer, comparative measurements using the roadblock mechanism in yeast were performed.

For a comparative measurement, the well-characterized and commonly used tetracycline aptamer, the neomycine aptamer and the new and high-performing tobramycine aptamer were chosen. All aptamers were cloned in the 5'UTR of a GFP reporter gene and the GFP expression was measured in yeast in the absence or presence of the respective ligand. For the tested ligand concentrations of 100 and 250 µM, the doxycycline-binding aptamer showed clearly the highest switching factor (cf. table 4, fig. 2c). This can be largely attributed to relatively high expression levels in the absence of ligand combined with comparable expression levels in the presence of ligand, when compared with the other aptamers. While the initially found aptamer G12 already outperformed the other aptamers, its variant S1 possessed the best regulatory behavior overall with a basal expression of 38 %, an expression level of 1,7 % at 250 µM doxycycline and a resulting switching factor of 25-fold. Since the regulation through the roadblock mechanism in yeast is greatly defined by the structural stability of the aptamer, the switching behavior of the doxycycline-binding aptamer suggests a great structural dynamic between the ligand-bound to unbound state.

### Example 4: Architecture and structural dynamics of the doxycycline-binding aptamer

Testing nucleotide flexibility through SHAPE-MaP provided insight into the structural nature and dynamic of the doxycycline-binding aptamer. SHAPE-Map was performed using the variant M10 in absence or presence of 100 µM doxycycline. Position reactivity in the absence of doxycycline supported overall the predicted 2D folding with the highest reactivity found in the loops and central junction, indicating high flexibility of the individual nucleotides in these areas as expected (fig. 3d). However, most nucleotides in the loop regions showed low reactivity which would be unexpected for unstructured regions, hinting towards tertiary structures formed by the loops, preventing nucleotide SHAPE reactivity. With the addition of doxycycline, high reactivity appeared at the same positions as observed without doxycycline, confirming the overall structure of the aptamer. When looking at the stabilizing effects of doxycycline on the aptamer structure, strong reduction in reactivity was observed in the P1, L2, P3 and L3 regions (fig. 3e). In case of the L2 and L3 region, the stabilized positions possessed low reactivity in the absence of doxycycline, further hinting towards a tertiary structure connected to ligand binding. The stabilization of the P1 and P3 stem are most likely not caused by ligand-aptamer interactions, since these regions can be mutated without affecting the regulatory capability of the aptamer. Together, these findings of SHAPE-MaP present a complex image of the structure of G12 and its interaction with doxycycline upon binding.

### Example 5: Stopped-flow spectroscopy (SF)

For obtaining insights into the aptamer's binding dynamics, stopped-flow spectroscopy (SF) was performed utilizing the intrinsic fluorescence of doxycycline caused by binding to the aptamer. Four measurements with increasing ligand concentration (2, 4, 6 & 8 equivalents) were conducted. The fluorescence increased with ligand binding and the binding process accelerated from 3 s to 1 s with higher equivalents of ligand. The binding mechanism of doxycycline to G12 was analyzed with a kinetic modelling (KM) approach. Starting with a simple one-step binding process, the model was progressively refined by incorporating additional conformational states, intermediate steps and reverse reactions. The comprehensive KM analysis is described in detail in the Supplementary Information. The best model of the KM depicted revealed two initial conformations of ligand free aptamer in equilibrium. Both conformations (R1 and R2) bind doxycycline via a two-step binding mechanism, involving an initial ligand-aptamer association followed by a ligand independent conformational rearrangement. The population plot based on this model indicates that binding primarily occurs through the binding pathway of the R2 and R2* conformations. The R1/R2 equilibrium rapidly shifts towards R2. The intermediate R1* is also formed, but its population remains negligible due to its fast rearrangement rate (kₑ) which is significantly faster than its formation rate (k_{c}).

These results suggest two aptamer conformations in equilibrium while one (R₂) facilitates faster ligand binding. However, in the second binding step its intermediate R₂* requires more extensive rearrangement towards the final bound conformation. In contrast, the conformation of R₁ slows down initial interaction with the ligand, while its intermediate R₁* allows for rapid conformational rearrangement.

The binding dynamic of G12 shares similarities with the tetracycline aptamer (Förster et al., 2011; doi:10.1093/nar/gkr835), both involving an initial equilibrium between two conformational states and a subsequent two-step ligand-induced rearrangement. The ligand-free equilibrium of the tetracycline aptamer depends on the binding of Mg²⁺-ions. Only the conformation which binds Mg²⁺ are ligand binding competent. The model for G12 indicates that the pathway via R₂ is favored in a similar fashion. Although melting curve analysis showed no dependency on Mg²⁺ for the structure of G12, fluorescence titration experiments revealed a dependency on Mg²⁺ for doxycycline binding. For both aptamers, ligand binding occurs preferentially in one of the two conformational states, followed by an intermediate state.

### Example 6: Application of the doxycycline-binding aptamer for gene regulation in human cells

As shown by the mutational, the binding motif of G12 seems to span from the P2 stem to the end of the P3 stem, leaving the P1 variable for riboswitch engineering. For the utilization of regulatory active aptamers in mammalian cells, control of mRNA processing has proven to be an efficient platform for gene regulation. The 3' splice site of an exon was placed under the control of a riboswitch, leading to incorporation or exclusion of the downstream exon based on the absence or presence of the ligand (fig. 2f). To measure the splice control, a luciferase dual reporter system was used (Firefly Luciferase und Renilla Luciferase), where the firefly luciferase was under control of the riboswitch and the renilla luciferase was used for normalization. The doxycycline binding motif of G12 was placed on an optimized stem for the tetracycline-binding aptamer to test for its capability in this system. When measured in comparison to the original construct the splice-regulator with G12 as its sensory domain performed slightly better than the original construct with the tetracycline-binding aptamer. In the context of splice regulation, G12 led to a reaction to lower ligand concentrations than the tetracycline aptamer and a higher switching factor at all tested concentrations (fig. 2g). Cross-reactivity between the aptamers and the ligands was tested, showing no signs of cross-reactivity, enabling orthogonal application of these switches in the same cell.

Another way to utilize regulatory active aptamers for the gene regulation in mammalian cells is the engineering of aptazymes. For the engineering of a new doxycycline-aptazyme, G12 was combined with the hammerhead ribozyme (HHR, fig. 2d). For initial tests, the two best performing P1 stems (K19, K4) of the previously engineered tetracycline-aptamzyme were used in combination with the doxycycline aptamer. In these constructed aptazymes, the rather loose aptamer structure in the absence of the ligand allows for correct formation of the ribozyme structure and thereby enabling ribozyme activity. Upon ligand binding, the aptamer structure rigidifies and prevents the correct structure of the ribozyme, thereby inhibiting ribozyme activity and subsequently keeping the controlled RNA intact. In the tested assay, the aptazymes were placed in the 3'UTR of a firefly luciferase gene thereby controlling the stability of the mRNA and ultimately controlling firefly luciferase expression. The luminescence of the firefly luciferase was then normalized to the luminescence of a constitutive expressed Renilla luciferase. Both tested stems (K19 and K4) showed a similar regulatory potential to the original K19-tetracycline-aptazyme construct. Although the expression levels of the G12 constructs were higher than with the tetracycline aptamer, the switching factors of 2,7- and 3,3-fold are comparable with the measured 2,9-fold of the tetracycline aptamer (fig. 2e).

In both *in vivo* applications described above, the doxycycline-binding aptamer G12 simply replaced another aptamer without further optimization of the constructs and still led to similar or better gene regulatory performance than with the original aptamer. Comparing this with the comparative aptamer measurements in yeast (fig. 2c), indicates that with an adapted sequence context the regulatory potential of the doxycycline-binding aptamer G12 can be even further improved. These data proof the applicability of the doxycycline-binding aptamer for *in vivo* gene regulation in a human context, enabling its use for the engineering of synthetic, mammalian riboswitches.

## Claims

**1.** A nucleic acid molecule comprising a sequence that undergoes a conformational change upon binding of doxycycline, wherein the nucleic acid molecule comprises a sequence selected from the group of sequences according to SEQ ID NO: 1 to SEQ ID NO: 29.

**2.** The nucleic acid molecule according to claim 1, wherein the nucleic acid molecule comprises a sequence selected from SEQ ID NO: 1 to SEQ ID NO: 26 or SEQ ID NO: 29.

**3.** The nucleic acid molecule according to claim 1, wherein the nucleic acid molecule comprises a sequence selected from SEQ ID NO: 1 to 3, or SEQ ID NO: 29.

**4.** The nucleic acid molecule according to claim 1, wherein the nucleic acid molecule comprises the sequence of SEQ ID NO: 3 or SEQ ID NO: 29.

**5.** The nucleic acid molecule of any of the previous claims, wherein the nucleic acid molecule further comprises a stem comprising a sequence selected from SEQ ID NO: 27 to 28.

**6.** The nucleic acid molecule of any of the previous claims, wherein the nucleic acid molecule binds to doxycycline with an affinity (K_{d}) of 20 nM or less.

**7.** The nucleic acid molecule of any of the previous claims, wherein the nucleic acid molecule does not bind to tetracycline as measured by ITC and/or fluorescence titration.

**8.** The nucleic acid molecule of any of the previous claims, wherein the nucleic acid molecule has a switching factor of at least 2.

**10.** The nucleic acid molecule of any of the previous claims, wherein the conformational change results in a gene-regulation selected from the group consisting of inhibition of transcription of a gene, activation of transcription of a gene, regulation of translation of a gene, regulating ribozyme activity, regulating nucleic acid hybridization, or affection of splicing of a gene, as well as combinations thereof.

**11.** Use of the nucleic acid molecule of any of the previous claims as part of a riboswitch, as part of an aptazyme, biosensor, targeted drug delivery system, molecular switches or devices for synthetic biology, or molecular memory systems.

**12.** Use of the nucleic acid molecule of any of claims 1 to 9 for gene-regulation selected from the group consisting of inhibition of transcription of a gene, activation of transcription of a gene, regulation of translation of a gene, or affection of splicing of a gene, as well as combinations thereof.

**13.** Use of the nucleic acid molecule of any of claims 1 to 9, wherein the molecule is combined with a second system for gene-regulation selected from the group comprising a Tet-On-system, CRISPR-Cas9-system, RNAi-system, ZINC Finger Nuclease (ZFN)-system, Transcription Activator-Like Effector Nucleases (TALENs)-system, further aptamer-systems, an optogenetics system, a heat-inducible system, a chemical-inducible system, and a protein dimerization system, as well as combinations thereof.

**14.** Pharmaceutical composition comprising the nucleic acid molecule of any of claims 1 to 9, further comprising at least one component selected from the group consisting of fillers, binders, disintegrants, lubricants, glidants, buffering agents, preservatives, stabilizers, coating agents, solvents, surfactants, and humectants, as well as combinations thereof.

**15.** The nucleic acid molecule of any of claims 1 to 9, or the pharmaceutical composition of claim 14, for use as a medicament.
